(19) Europäisches Patentamt
European Patent Office
Office européen des brevets

(11) Veröffentlichungsnummer : **0 114 289**
**B1**

# (12) EUROPÄISCHE PATENTSCHRIFT

(45) Veröffentlichungstag der Patentschrift : **20.08.86**

(51) Int. Cl.[4] : **C 07 D239/36**, C 07 D239/52, C 07 D239/70

(21) Anmeldenummer : **83112468.0**

(22) Anmeldetag : **12.12.83**

## (54) Verfahren zur Herstellung von 2-Alkylthiomethyl-4-hydroxypyrimidinen.

(30) Priorität : **24.12.82 DE 3247926**

(43) Veröffentlichungstag der Anmeldung : **01.08.84 Patentblatt 84/31**

(45) Bekanntmachung des Hinweises auf die Patenterteilung : **20.08.86 Patentblatt 86/34**

(84) Benannte Vertragsstaaten : **BE CH DE FR GB LI**

(56) Entgegenhaltungen : **EP-A- 0 022 511**
**GB-A- 2 083 814**

(73) Patentinhaber : **BAYER AG**
**Konzernverwaltung RP Patentabteilung**
**D-5090 Leverkusen 1 Bayerwerk (DE)**

(72) Erfinder : **Maurer, Fritz, Dr.**
**Roeberstrasse 8**
**D-5600 Wuppertal 1 (DE)**

**EP 0 114 289 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von 2-Alkylthiomethyl-4-hydroxypyrimidinen.

Es ist bereits bekannt, daß man 2-Alkylthiomethyl-4-hydroxypyrimidine erhält, wenn man 2-Chlormethyl-4-hydroxypyrimidine mit Alkalithiolaten umsetzt (vgl. DE-OS 2 752 613 bzw. US-PS 4 200 637). Der Nachteil dieses Verfahrens besteht darin, daß die Ausgangsverbindung 2-Chlormethyl-4-hydroxypyrimidin nur in aufwendiger Synthese über 4 Stunfen erhältlich ist (C.A., *71*, 70 563a (1969)) und daß die Ausbeuten an 2-Alkylthiomethyl-4-hydroxypyrimidin häufig sehr unbefriedigend sind (vgl. DE-OS 2 752 613 und DE-OS 2 838 359).

Weiterhin ist bekannt, daß man 2-Alkylthiomethyl-4-hydroxypyrimidine auch erhält, wenn man Alkylthioacetamidine mit β-Ketocarbonsäureestern cyclisiert (vgl. EP-OS 22 511). Nachteilig bei diesem Verfahren ist jedoch, daß die als Ausgangsprodukte verwendeten Amidine nur in einer mehrstufigen Reaktion aus Chloracetonitril erhältlich sind (Houben Weyl, Methoden der organischen Chemie, Band 8, 4. Auflage, Thieme Verlag Stuttgart, 1952, S. 698, 699, 702), wodurch das Verfahren mit relativ schlechten Ausbeuten abläuft.

« Aus GB-A-2 083 814 ist ein Verfahren zur Herstellung von 2-Isopropyl-4-methyl-6-hydroxypyrimidin bekannt, welches die Umsetzung eines Iminoisobutyrats mit Ammoniak in Methanol oder Ethanol und gegebenenfalls Wasser und die Umsetzung des entstehenden Amidins in dem bestimmten Alkanol mit Alkylacetacetat betrifft. »

Es wurde gefunden, daß man 2-Alkylthiomethyl-4-hydroxypyrimidine der Formel (I)

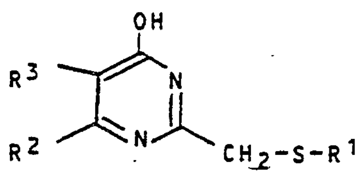

(I)

in welcher

$R^1$ für Alkyl steht,

$R^2$ für Wasserstoff oder Alkyl steht,

$R_3$ für Wasserstoff, Alkyl oder Alkoxy steht oder

$R^2$ und $R^3$ zusammen für einen zweifach gebundenen Alkylenrest stehen,

in einem mehrstufigen Verfahren ohne Isolierung der Zwischenstufen erhält, wenn man Sulfonyloxyacetonitrile der allgemeinen Formel (II)

$$R{-}SO_2{-}O{-}CH_2{-}CN \qquad (II)$$

in welcher

R für Alkyl, Halogenalkyl oder gegebenenfalls substituiertes Aryl steht,

gegebenenfalls in Gegenwart eines Verdünnungsmittels sowie gegebenenfalls in Gegenwart katalytischer Mengen einer Base mit Methanol oder Ethanol umsetzt, das entstehende Reaktionsgemisch mit Ammoniumchlorid umsetzt, das entstehende Reaktionsgemisch mit Alkalithiolat der Formel (III)

$$R^1{-}S^{\ominus}Me^{\oplus} \qquad (III)$$

in welcher

$R^1$ die oben angegebene Bedeutung hat und

$Me^{\oplus}$ für ein Alkalimetallkation steht,

und in Gegenwart einer Base mit β-Ketocarbonsäureester der Formel (IV)

$$R^2{-}CO{-}\underset{\displaystyle R^3}{\underset{|}{CH}}{-}CO{-}O{-}R^1 \qquad (IV)$$

in welcher

$R^2$ und $R^3$ die oben angegebene Bedeutung haben und

$R^1$ für Alkyl steht,

bei Temperaturen zwischen — 20 und + 100 °C umsetzt.

Es ist als ausgesprochen überraschend anzusehen, daß man mit Hilfe des erfindungsgemäßen Verfahrens die gewünschten 2-Alkylthiomethyl-4-hydroxypyrimidine in guten Ausbeuten erhält, da die aus dem Stand der Technik bekannten einzelnen Stufen bei der Herstellung der 2-Alkylthiomethyl-4-hydroxypyrimidine in der Regel nur unbefriedigende Ausbeuten ergeben. Es konnte nicht erwartet werden, daß die 2-Alkylthiomethyl-4-hydroxypyrimidine über 4 Stunfen ohne Isolierung und Reinigung der Zwischenstufen in guter Ausbeute und Reinheit erhalten werden.

Mit Hilfe des erfindungsgemäßen Verfahrens erhält man vorzugsweise solche Verbindungen der Formel (I), bei denen

$R^1$ für geradkettiges oder verzweigtes Alkyl mit bis zu 6, insbesondere mit 1 bis 4, Kohlenstoffatomen steht und

$R^2$ für Wasserstoff oder für geradkettiges oder verzweigtes Alkyl mit bis zu 6, insbesondere mit 1 bis 4, Kohlenstoffatomen steht und

$R^3$ für Wasserstoff, für geradkettiges oder verzweigtes Alkyl mit bis zu 6, insbesondere mit 1 bis 4, Kohlenstoffatomen oder für geradkettiges oder verzweigtes Alkoxy mit bis zu 6, insbesondere mit 1 bis 4, Kohlenstoffatomen steht oder

$R^1$ und $R^2$ zusammen für einen zweifach gebundenen Alkylenrest mit 3 bis 5 Kohlenstoffatomen stehen.

Verwendet man beispielsweise p-Toluolsulfonsäurecyanmethylester, Natrium-Methylthiolat und 2-Methylacetessigsäuremethylester als Ausgangsstoffe, und Natriummethylat als Base, so läßt sich das erfindungsgemäße Verfahren durch das folgende Formelschema darstellen :

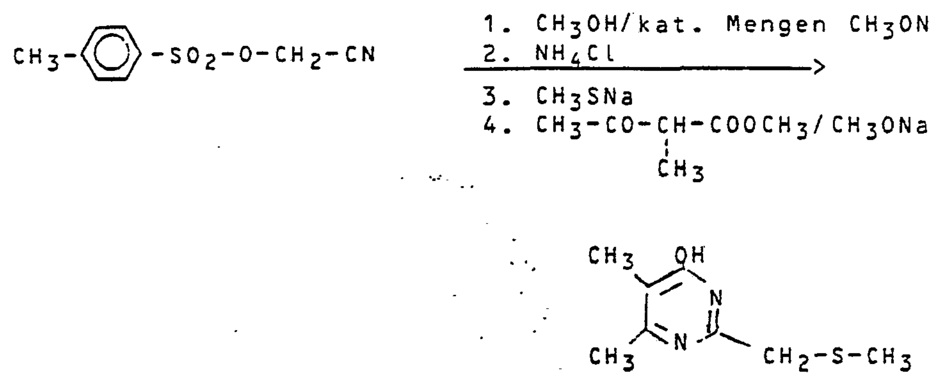


Die zur Durchführung des erfindungsgemäßen Verfahrens als Ausgangsstoffe benötigten Sulfonyloxyacetonitrile sind durch die Formel (II) allgemein definiert. In dieser Formel steht R vorzugsweise für geradkettiges oder verzweigtes Alkyl mit bis zu 4 Kohlenstoffatomen, für geradkettiges oder verzweigtes Halogenalkyl mit bis zu 10 Kohlenstoffatomen und bis zu 21 Halogenatomen, oder für gegebenenfalls ein- bis dreifach gleich oder verschieden durch Halogen, Nitro, Cyano, Alkyl oder Alkoxy mit je bis zu 4 Kohlenstoffatomen oder Halogenalkyl mit bis zu 2 Kohlenstoffatomen und bis zu 5 Halogenatomen substituiertes Phenyl. Besonders bevorzugt steht R für Phenyl, p-Tolyl, Methyl, Trifluormethyl oder Nonafluorbutyl. Die Sulfonyloxyacetonitrile der Formel (II) sind bekannt oder lassen sich nach bekannten Verfahren in einfacher Weise herstellen, indem man Sulfonsäurechloride der Formel (V)

$$R{-}SO_2{-}Cl \qquad (V)$$

in welcher R die oben angegebene Bedeutung hat,
mit Formalin und Natriumcyanid umsetzt (vgl. Bull. Soc. Chim. France *1948*, 945).

Die weiterhin zur Durchführung des erfindungsgemäßen Verfahrens als Ausgangsstoffe benötigten Alkalithiolate sind durch die Formel (III) allgemein definiert. In dieser Formel steht $R^1$ vorzugsweise für geradkettiges oder verzweigtes Alkyl mit bis zu 6, insbesondere mit 1 bis 4, Kohlenstoffatomen, $Me^{\oplus}$ steht vorzugsweise für ein Natrium- oder Kaliumkation. Die Alkalithiolate der Formel (III) sind allgemein bekannte Verbindungen der organischen Chemie.

Die weiterhin zur Durchführung des erfindungsgemäßen Verfahrens als Ausgangsstoffe benötigten β-Ketocarbonsäureester sind durch die Formel (IV) allgemein definiert. In dieser Formel stehen $R^2$ und $R^3$ vorzugsweise für diejenigen Reste, die schon bei der Beschreibung der entsprechenden Reste der Verbindungen der Formel (I) als bevorzugt aufgezählt wurden. $R^1$ steht vorzugsweise für geradkettiges oder verzweigtes Alkyl mit bis zu 4 Kohlenstoffatomen. Die β-Ketocarbonsäureester der Formel (IV) sind ebenfalls allgemein bekannte Verbindungen der organischen Chemie.

Das erfindungsgemäße Verfahren wird vorzugsweise in Gegenwart eines geeigneten Verdünnungsmittels durchgeführt. Als solche kommen praktisch alle inerten organischen Lösungsmittel in Frage.

Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Ether wie Diethyl- und Dibutylether, Tetrahydrofuran und Dioxan, sowie Alkohole, wie Methanol, Ethanol und Isopropanol. Bevorzugt sind Alkohole, insbesondere Methanol und Ethanol.

Das erfindungsgemäße Verfahren kann gegebenenfalls in Gegenwart von Basen durchgeführt werden. Als Basen können alle üblichen organischen oder anorganischen Basen verwendet werden.

Besonders bewährt haben sich Alkalicarbonate und -alkoholate, wie Natrium- und Kaliumcarbonat, Natrium- und Kaliummethylat bzw. -ethylat, ferner aliphatische, aromatische und heterocyclische Amine,

beispielsweise Triethylamin, Trimethylamin, Dimethylanilin, Dimethylbenzylamin und Pyridin. Bevorzugt sind Alkalialkoholate, insbesondere Alkalimethylate und -ethylate.

Das erfindungsgemäße Verfahren wird im allgemeinen bei Temperaturen zwischen —20 und + 100 °C durchgeführt. Bevorzugt ist der Temperaturbereich zwischen 0 und + 50 °C, insbesondere zwischen 0 °C und Raumtemperatur. Die Umsetzungen werden im allgemeinen bei Normaldruck durchgeführt.

Das Verfahren wird durchgeführt, indem man zunächst die Sulfonyloxyacetonitrole der Formel (II) mit äquimolaren Mengen Methanol oder Ethanol umsetzt. Bevorzugt wird dabei Methanol oder Ethanol als Lösungsmittel im Überschuß eingesetzt. Die Gegenwart katalytischer Mengen einer Base hat sich als vorteilhaft erwiesen.

Anschließend wird das Reaktionsgemisch mit Ammoniumchlorid versetzt. Bezogen auf Sulfonyloxyacetonitril werden 1-2 Mol, bevorzugt 1-1,2 Mol, Ammoniumchlorid eingesetzt.

Nachdem sich das bei diesen Reaktionen entstandene Hydrochlorid des Sulfonyloxacetamidins gebildet hat wird das Reaktionsgemisch entweder zuerst mit Alkalithiolat und dann in Gegenwart einer Base mit einem β-Ketocarbonsäureester umgesetzt oder zuerst in Gegenwart einer Base mit dem β-Ketocarbonsäureester und dann mit den Alkalithiolat umgesetzt. Bevorzugt wird jedoch das Reaktionsgemisch zuerst mit dem Alkalithiolat und dann mit dem β-Ketocarbonsäureester umgesetzt.

Das Alkalithiolat kann vorteilhafterweise *in situ* gebildet werden, indem man das Reaktionsgemisch zunächst mit dem entsprechenden Merkaptan (1-1,5 Mol pro Mol Sulfonyloxyacetonitril) versetzt und anschließend die entsprechende Menge einer Base zugibt.

Die Reaktion mit dem β-Ketoester erfolgt in Gegenwart einer Base. Es werden 2-2,5, bevorzugt 2-2,2 Mol Base pro Mol Sulfonyloxyacetonitril eingesetzt.

Bei Verwendung von Verdünnungsmitteln werden diese nach Reaktionsende im Vakuum abdestilliert. Der Rückstand wird dann in Wasser gelöst, die Lösung durch Zugabe einer Säure wie z. B. Salzsäure, Schwefelsäure, Phosphorsäure, Ameisensäure oder Essigsäure neutralisiert und das ausgefallene produkt abgesaugt. Für den Fall, daß die Reaktionsprodukte in Wasser löslich sind, werden sie durch Extraktion der wäßrigen Lösung mit einem mit Wasser nicht mischbaren organischen Lösungsmittel, wie z. B. Chlorkohlenwasserstoffen oder niedrigen Carbonsäurealkylestern, isoliert.

Die Verbindungen der Formel (I) fallen in der Regel in fester Form an und können durch Umkristallisation gereinigt werden. Zur Charakterisierung dient der Schmelzpunkt.

Die mit Hilfe des erfindungsgemäßen Verfahrens hergestellten 2-Alkylthiomethyl-4-hydroxypyrimidine dienen als Ausgangsstoffe für hochwirksame Insektizide (vgl. EP-A-22 511 und DE-A-2 752 613 bzw. US-A-4 200 637).

Herstellungsbeispiele

Beispiel 1

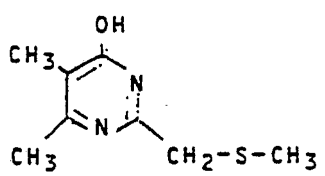

Zu einer Lösung von 0,01 Mol Natriummethylat in 100 ml Methanol gibt man bei 0 bis 5 °C portionsweise 21,1 g (0,1 Mol) p-Toluolsulfonsäure-cyanmethylester. Man läßt das Gemisch auf 20 °C kommen, gibt bei dieser Temperatur 5,9 g (0,11 Mol) Ammoniumchlorid zu und rührt 4 Stunden bei Raumtemperatur. Dann leitet man bei 10 °C 6 g (0,125 Mol) Methylmercaptan ein, tropft bei der gleichen Temperatur eine Lösung von 0,11 Mol Natriummethylat in 20 ml Methanol zu und rührt erst 1 Stunde bei 0 bis 5 °C, dann 2 Stunden ohne Kühlung nach. Das Reaktionsgemisch wird bei Raumtemperatur mit weiteren 0,22 Mol Natriummethylat in 40 ml Methanol und dann mit 13 g (0,1 Mol) 2-Methylacetessigsäure-methylester versetzt und weitere 18 Stunden bei Raumtemperatur nachgerührt. Anschließend wird dfas Lösungsmittel im Vakuum abdestilliert, der Rückstand in 100 ml Wasser gelöst und bei 10 bis 20 °C mit konzentrierter Salzsäure auf pH 4 eingestellt. Das ausgefallene Produkt wird abgesaugt und mit 20 ml kaltem Wasser nachgewaschen. Man erhält auf diese Weise 12,8 g (70 % der Theorie) 2-Methylthiomethyl-4-hydroxy-5,6-dimethylpyrimidin in Form eines beigen Pulvers mit dem Schmelzpunkt 166 °C.

Beispiel 2

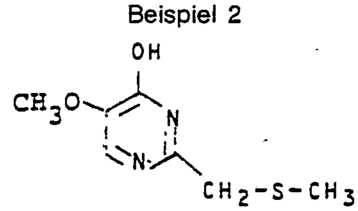

Eine Lösung von 0,01 Mol Natriummethylat in 100 ml Methanol wird bei 0 bis 5 °C mit 21,1 g (0,1 Mol) p-Toluolsulfonsäure-cyanmethylester versetzt. Man läßt das Gemisch auf 20 °C kommen, gibt bei dieser Temperatur 5,9 g (0,11 Mol) Ammoniumchlorid zu und rührt 4 Stunden bei Raumtemperatur nach. Dann leitet man bei 10 °C 6 g (0,125 Mol) Methylmercaptan ein, tropft bei der gleichen Temperatur eine Lösung von 0,11 Mol Natriummethylat in 20 ml Methanol zu und rührt erst 1 Stunde bei 0 bis 5 °C, dann 2 Stunden ohne Kühlung nach. Das Reaktionsgemisch wird bei Raumtemperatur mit weiteren 0,11 Mol Natriummethylat in 20 ml Methanol und dann mit 15,4 g (0,1 Mol) Hydroxymethylen-methoxyessigsäuremethylester-Natriumsalz versetzt und weitere 18 Stunden bei Raumtemperatur nachgerührt. Anschließend wird das Lösungsmittel im Vakuum abdestilliert, der Rückstand in 100 ml Wasser gelöst und bei 10 bis 20 °C mit konzentrierter Salzsäure auf pH 4 eingestellt. Dann extrahiert man dreimal mit je 100 ml Methylenchlorid, trocknet die organische Phase über Natriumsulfat und destilliert das Lösungsmittel im Vakuum ab. Zurück bleiben 11,1 g (60 % der Theorie) 2-Methylthiomethyl-4-hydroxy-5-methoxypyrimidin in Form eines beigen Pulvers mit dem Schmelzpunkt 136 °C.

Analog Beispiel 1 oder 2 können z. B. die folgenden Verbindungen hergestellt werden :

| | Schmelzpunkt (°C) |
|---|---|
| 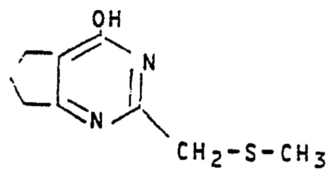 | 170 |
| 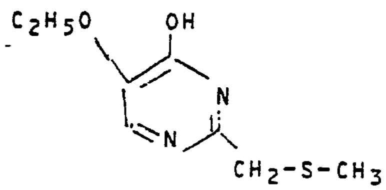 | 177 (Z) |
| 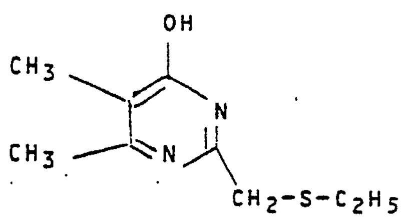 | 122 |
| 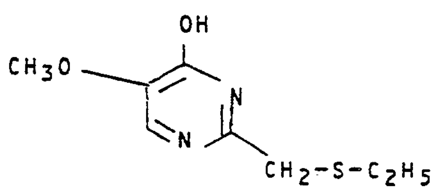 | 151 |
| 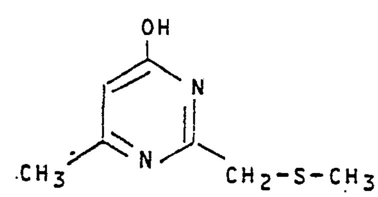 | 138 |
| 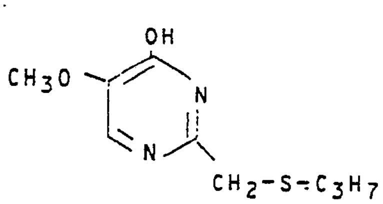 | 119 |

**Patentansprüche**

1. Verfahren zur Herstellung von 2-Alkylthiomethyl-4-hydroxypyrimidinen der Formel (I)

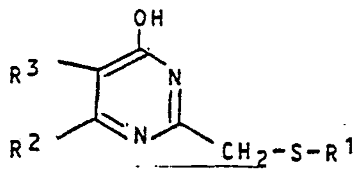

(I)

in welcher
$R^1$ für Alkyl steht,
$R^2$ für Wasserstoff oder Alkyl steht,
$R^3$ für Wasserstoff, Alkyl oder Alkoxy steht oder
$R^2$ und $R^3$ zusammen für einen zweifach gebundenen Alkylenrest stehen,
in einem mehrstufigen Verfahren ohne Isolierung der Zwischenstufen, dadurch gekennzeichnet, daß man Sulfonyloxyacetonitrile der allgemeinen Formel (II)

$$R—SO_2—O—CH_2—CN \qquad (II)$$

in welcher R für Alkyl, Halogenalkyl oder gegebenenfalls substituiertes Aryl steht, gegebenenfalls in Gegenwart eines Verdünnungsmittels sowie gegebenenfalls in Gegenwart katalytischer Mengen einer Base mit Methanol oder Ethanol umsetzt, das entstehende Reaktionsgemisch mit Ammoniumchlorid umsetzt, das entstehende Reaktionsgemisch mit Alkalithiolat der Formel (III)

$$R^1—S^{\ominus}Me^{\oplus} \qquad (III)$$

in welcher
$R^1$ die oben angegebene Bedeutung hat und
$Me^{\oplus}$ für ein Alkalimetallkation steht,
und in Gegenwart einer Base mit β-Ketocarbonsäureester der Formel (IV)

$$R^2-CO-\underset{R^3}{CH}-CO-O-R^1 \qquad (IV)$$

in welcher
$R^2$ und $R^3$ die oben angegebene Bedeutung haben und
$R^1$ für Alkyl steht,
bei Temperaturen zwischen —20 und + 100 °C umsetzt.

2. Verfahren gemäß Anspruch 1, dadurch gekennzeichnet, daß in Methanol oder Ethanol als Lösungsmittel gearbeitet wird.

3. Verfahren gemäß Anspruch 1, dadurch gekennzeichnet, daß das Alkalithiolat der Formel (III) *in situ* hergestellt wird.

4. Verfahren gemäß Anspruch 1, dadurch gekennzeichnet, daß als Base Alkalialkoholat eingesetzt wird.

5. Verfahren gemäß Anspruch 1, dadurch gekennzeichnet, daß bei Temperaturen zwischen 0 °C und Raumtemperatur gearbeitet wird.

**Claims**

1. Process for the preparation of 2-alkylthiomethyl-4-hydroxypyrimidines of the formula (I)

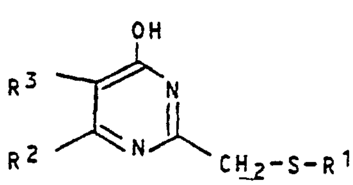

(I)

in which
$R^1$ represents alkyl,

$R^2$ represents hydrogen or alkyl and
$R^3$ represents hydrogen, alkyl or alkoxy, or
$R^2$ and $R^3$ together represent a divalent alkylen radical,
in a multi-stage process without isolation of the intermediates, characterised in that sulphonyloxyacetonitriles of the general formula (II)

$$R-SO_2-O-CH_2-CN \qquad (II)$$

in which R represents alkyl, halogenoalkyl or optionally substituted aryl, are reacted with methanol or ethanol, if appropriate in the presence of a diluent and if appropriate in the presence of catalytic amounts of a base, the reaction mixture formed is reacted with ammonium chloride, and the reaction mixture formed is reacted with an alkali metal thiolate of the formula (III)

$$R^1-S^{\ominus}Me^{\oplus} \qquad (III)$$

in which
$R^1$ has the abovementioned meaning and
$Me^{\oplus}$ represents an alkali metal cation, and, in the presence of a base, with a β-ketocarboxylic acid ester of the formula (IV)

$$R^2-CO-\underset{R^3}{\underset{|}{CH}}-CO-O-R^1 \qquad (IV)$$

in which
$R^2$ and $R^3$ have the abovementioned meaning and
$R^1$ represents alkyl,
at temperatures between —20 and + 100 °C.

2. Process according to Claim 1, characterised in that it is carried out in methanol or ethanol, as the solvent.

3. Process according to Claim 1, characterised in that the alkali metal thiolate of the formula (III) is prepared *in situ*.

4. Process according to Claim 1, characterised in that an alkali metal alcoholate is used as the base.

5. Process according to Claim 1, characterised in that it is carried out at temperatures between 0 °C and room temperature.

**Revendications**

1. Procédé de préparation de 2-alkylthiométhyl-4-hydroxypyrimidines de formule (I) :

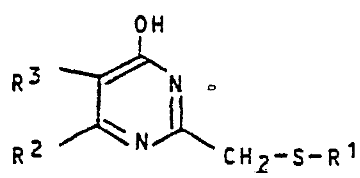

(I)

dans laquelle :
$R^1$ représente un groupe alkyle,
$R^2$ représente l'hydrogène ou un groupe alkyle,
$R^3$ représente l'hydrogène, un groupe alkyle ou alcoxy, ou bien $R^2$ et $R^3$ forment ensemble un groupe alkylène à deux liaisons,
dans un procédé à plusieurs stades opératoires sans isolement des produits intermédiaires, caractérisé en ce que l'on fait réagir des sulfonyloxyacétonitriles de formule générale (II) :

$$R-SO_2-O-CH_2-CN \qquad (II)$$

dans laquelle R représente un groupe alkyle, halogénoalkyle ou aryle éventuellement substitué, éventuellement en présence d'un diluant et éventuellement en présence de quantités catalytiques d'une base, avec le méthanol ou l'éthanol, on fait réagir le mélange de réaction formé avec du chlorure d'ammonium, on fait réagir le mélange de réaction formé avec un thiolate alcalin de formule (III) :

$$R^1-S^{\ominus}Me^{\oplus} \qquad (III)$$

dans laquelle :

$R^1$ a les significations indiquées ci-dessus, et

$Me^+$ représente un cation de métal alcalin,

et en présence d'une base, avec un ester d'acide β-cétocarboxylique de formule (IV) :

$$R^2\text{-CO-}\underset{R^3}{\underset{|}{CH}}\text{-CO-O-R}^1 \qquad \text{(IV)}$$

dans laquelle :

$R^2$ et $R^3$ ont les significations indiquées ci-dessus, et

$R^1$ représente un groupe alkyle,

à des températures de — 20 à + 100 °C.

2. Procédé selon la revendication 1, caractérisé en ce que l'on opère dans le méthanol ou l'éthanol servant de solvant.

3. Procédé selon la revendication 1, caractérisé en ce que l'on prépare le thiolate alcalin de formule (III) *in situ*.

4. Procédé selon la revendication 1, caractérisé en ce que la base utilisée est un alcoolate alcalin.

5. Procédé selon la revendication 1, caractérisé en ce que l'on opère à des températures comprises entre 0 °C et la température ambiante.